# EUROPEAN PATENT SPECIFICATION

(11) **EP 1 477 131 B1**
(45) Date of publication and mention of the grant of the patent: **16.09.2009**
(21) Application number: 04010120.6
(22) Date of filing: 28.04.2004
(51) Int. Cl.: A61F 2/06

(54) **Protective elongated sleeve for stent systems**
Schutzhülle für Stentsysteme
Manchon de protection alongé pour systèmes de stents

(30) Priority: 01.05.2003 US 466935 P
(43) Date of publication of application: 17.11.2004
(73) Proprietor: Medtronic Vascular, Inc., Santa Rosa, CA 95403 (US)
(72) Inventor: Cervantes, Marvin J., Edmonton, Alberta T5T3R6 (CA)
(74) Representative: Hughes, Andrea Michelle

(56) References cited:
- EP-A1- 0 796 633
- EP-A2- 0 819 413
- EP-A2- 1 025 813
- EP-A2- 1 053 722
- US-A1- 2002 116 046

## Description

### FIELD OF THE INVENTION

This invention relates generally to catheter deployment of drug-coated stents. More specifically, the invention relates to a protective elongated sleeve for stent systems.

### BACKGROUND OF THE INVENTION

An increasing number of stents for treating vascular conditions are being coated with pharmaceutical drugs and protective materials for controlled time-delivery of the therapeutic agents. Medical research indicates a greater effectiveness of vascular stents when the stents are coated with pharmaceutical drugs that help prevent or treat medical conditions. These drugs may be released from a coating while in the body, delivering their patent effects at the site where they are most needed. The drugs may be mixed, for example, within drug-polymers or encased by polymeric coatings on the stents. Stent coatings with various families of drug polymer chemistries have been used to increase the effectiveness of stenting procedures and to control drug-elution properties.

Unfortunately, drug-coated stents, and all other stents, are somewhat fragile, and deployment may lead to several undesirable situations. While inserting the stent through the Y-arm, the stent may contact the inner surfaces of the Y-arm, and may even rub against the o-ring in the toughy. In the case of a drug-eluting stent, such contact may disturb the polymeric surface, and reduce the effectiveness of the covering. While passing through the vasculature, the surface of the stent may also encounter resistance and contact the sides. Such contact may result in the loss of desired effects at the target site, as the drug elutes through the impacted surface.

EP 1053722 provides a low profile stenting system that has a sheath with an ultra-thin distal section that is shrunk down over the stent and over the distal tip of the catheter.

It is desirable, therefore, to provide a device that overcomes these and other disadvantages.

### SUMMARY OF THE INVENTION

in The subject-matter according to the present invention provides a system for treating a vascular condition comprising a catheter, a stent deployment assembly coupled to the catheter; the stent deployment assembly comprising a coated stent including a stent framework and a drug coating disposed on at least a portion of the stent framework; and a protective sleeve removably covering the stent deployment assembly and at least a portion of the catheter, wherein said sleeve comprises a hollow tube having a proximal outer diameter, a medial inner diameter, and a distal inner diameter; and wherein the distal inner diameter is sufficient to encircle an outer diameter of the stent deployment assembly, wherein the medial inner diameter is sufficient to encircle an outer diameter of the catheter, wherein the distal inner diameter is open, and wherein the protective sleeve is removed from covering the stent framework prior to deploying the stent; and characterized by a port to a vessel, the port including an O-ring having an O-ring inner diameter, wherein a proximal portion of the sleeve is positioned proximal to the port vessel and wherein the outer diameter of the proximal portion is greater than the O-ring diameter.

The present invention is illustrated by the accompanying drawings of various embodiments and the detailed description given below. The drawings should not be taken to limit the invention to the specific embodiments, but are for explanation and understanding. The detailed description and drawings are merely illustrative of the invention rather than limiting, the scope of the invention being defined by the appended claims. The foregoing aspects and other attendant advantages of the present invention will become more readily appreciated by the detailed description taken in conjunction with the accompanying drawings.

### BRIEF DESCRIPTION OF THE DRAWINGS

FIG. 1 is an illustration of a system for treating a vascular condition, in accordance with one embodiment of the current invention;
FIG. 2 is a cross-sectional view of a sent deployment assembly, in accordance with one embodiment of the current invention;
FIG. 3 is a side view of a protective sleeve, in accordance with one embodiment of the current invention;
FIG. 4 is an illustration of a system for treating a vascular condition;
FIG. 5 is an illustration of a system for treating a vascular condition;
FIG. 6 is an illustration of a system for treating a vascular condition;
FIG. 6A is close up view of the distal end of the system illustrated in FIG. 6;
FIG. 7 is an illustration of a system for treating a vascular condition;
FIG. 8A is an illustration of a system for treating a vascular condition;
FIG 8B is a close up view of the distal end of the system illustrated in FIG. 8A;
   and
FIG. 9 is a cross-section of a protective sleeve in accordance with one aspect of the invention.

### DETAILED DESCRIPTION OF THE PRESENTLY PREFERRED EMBODIMENTS

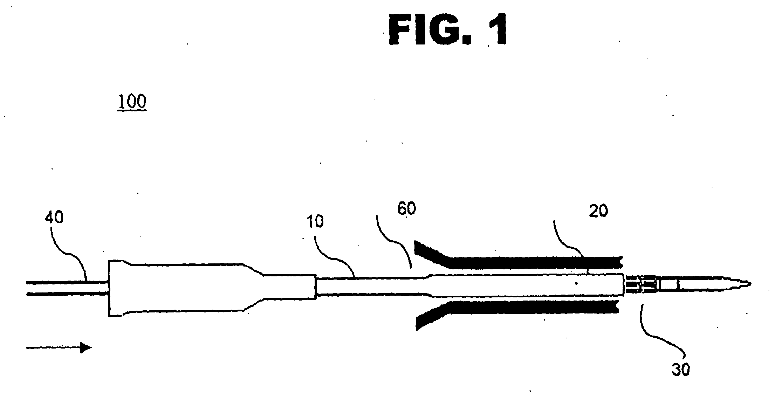

FIG. 1 shows one embodiment of the stent assembly 100. Stent assembly 100 comprises a catheter 40, a protective sleeve 10, a y-arm port 60, and a stent 20. FIG. 1 shows the sleeve 10 partially disposed within a leg of a Y-arm port 60. Y-arm ports are well known to those of ordinary skill in the art. In the embodiments of the present invention, the sleeve 10 is longer than the length of the Y-arm.

In each of the embodiments disclosed herein, the stent includes a stent framework and may comprise a drug coating on at least a portion of the stent framework. The stent framework may comprise a polymeric base or a metallic base such as stainless steel, nitinol, tantalum, MP35N alloy, platinum, titanium, a suitable biocompatible alloy, a suitable biocompatible material, a suitable polymeric material, or a combination thereof. The polymeric base material may comprise any suitable polymer for biomedical stent applications, as is known in the art. In other embodiments, the stent framework may comprise a dissolvable material, such that the stent framework dissolves while implanted in a vessel of a body.

The drug coating may include or encapsulate one or more therapeutic agents. The drug coating may comprise one or more therapeutic agents dispersed within or encased by a polymeric coating, which are eluted from the coated stent with controlled time delivery after deployment of coated stent within a body. A therapeutic agent is capable of producing a beneficial effect against one or more conditions including coronary restenosis, cardiovascular restenosis, angiographic restenosis, arteriosclerosis, hyperplasia, and other diseases and conditions. For example, the therapeutic agent can be selected to inhibit or prevent vascular restenosis, a condition corresponding to a narrowing or constriction of the diameter of the bodily lumen where the stent is placed. The drug coating may comprise, for example, an antirestenotic drug, an antisense agent, an antineoplastic agent, an antiproliferative agent, an antithrombogenic agent, an anticoagulant, an antiplatelet agent, an antibiotic, an anti-inflammatory agent, a steroid, a gene therapy agent, an organic drug, a pharmaceutical compound, a recombinant DNA product, a recombinant RNA product, a collagen, a collagenic derivative, a protein, a protein analog, a saccharide, a saccharide derivative, a bioactive agent, a pharmaceutical drug, a therapeutic substance, or combinations thereof. The elution rates of the therapeutic agents into the body and the tissue bed surrounding the stent framework are based on the constituency and thickness of drug-polymer coating, the nature and concentration of the therapeutic agents, the thickness and composition of cap coat, and other factors.

Protective sleeve 10 is slidably disposed around catheter 40, and stent 20. In one embodiment, sleeve 10 is fabricated of clear material to allow for viewing a stent 20 contained within the sleeve 10. Alternatively, the sleeve 10 may be fabricated of opaque material to aid in preventing degradation of the coating 30 from light. Alternatively, sleeve 10 may be fabricated of ultraviolet-filtering material for protection from UV radiation. The sleeve 10 may be made, for example, of Teflon or other suitable materials. In other embodiments, sleeve 10 may be made from nylon, polyurethane, polyethylene terephthalate, polyethylene, polytetrafluoroethylene, expanded polytetrafluoroethylene, an elastane, a thermoplastic elastomer, a woven polymeric fabric, or an expandable polymeric sheet.

Drug coating 30 may include one or more drugs, and may comprise a drug-polymer coating. Each drug may include a bioactive agent. The bioactive agent may be a pharmacologically active drug or bioactive compound. The bioactive agent may be eluted from the drug coating 30 when the stent 20 has been deployed in the body. Elution refers to the transfer of the bioactive agent out from drug coating 30. The elution rate is determined by the rate at which the bioactive agent is excreted from drug-polymer coating 30 into the body. The composition of the drug-polymer coating and the interdispersed drugs may control the elution rate of the bioactive agent. The phenoxy primer coating underlying drug coating 30 would tend not to be eluted, metabolized, or discarded by the body.

The drug coating 30 may be subject to degradation during processing, packaging, sterilization, or storage of a drug-polymer coated stent. During sterilization, for example, oxidation of the drug or polymer may occur, resulting in hydrolytic damage, cleavage of the polymeric bonds, breakdown of the polymer and/or drug, or actual cracking or peeling of the drug-polymer coating. Temperature excursions of the in-process or processed stent 20 may incite delamination of all or a portion of the drug coating 30. For stents comprising a dissolvable material, the risks of degradation are increased because of the lack of a metallic structure. The present invention solves this problem through the use of a protective sleeve to reduce or prevent drug-polymer delamination.

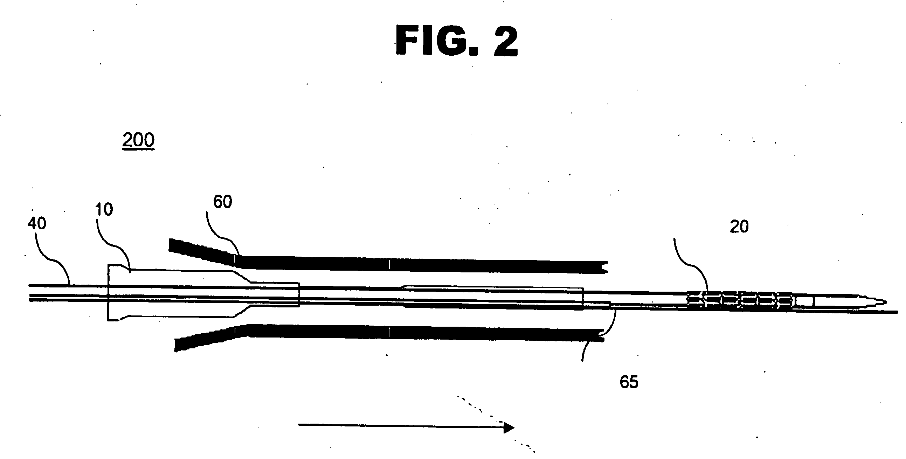

FIG. 2 illustrates stent assembly 200. Stent assembly 200 comprises a catheter 40, a protective sleeve 10, a y-arm port 60, and a stent 20. Stent 20 may comprise a drug coating 30. As seen in FIG. 2, sleeve 10 protects stent 20 from manual handling as stent 20 may be threaded out of sleeve 10 by deploying catheter 40 in the direction of the target site (indicated by arrow). Catheter 40 may protrude distally beyond sleeve 10 thereby providing a means for manipulating stent 20 without handling the stent within sleeve 10. When stent segment 20 is disposed within sleeve 10, sleeve 10 may be handled without handling stent segment 20. In FIG. 1, stent 20 is illustrated partially disposed within the protective sleeve; while in FIG. 2, stent 20 is illustrated not disposed within the protective sleeve 10. Stent segment 20 is therefore, less likely to be disturbed during storage, handling or sleeve placement. Furthermore, sleeve 10 may be handled without disturbing a coating 30 that may be dispersed on stent segment 20. Coating 30 is therefore, less likely to be disturbed during storage, sleeve placement and during lab handling. In embodiments where the stent 20 comprises a dissolvable material, protective sleeve 10 contributes to handling the stent with minimal contact with the actual stent 20.

FIG. 2 shows the sleeve 10 disposed within the Y-arm port 60. In this embodiment, the sleeve 10 will pass through the Y-arm port 60 and will protect the stent 20 until it is removed when the stent 20 is at the desired position. In the embodiments of the present invention, the outer diameter of sleeve 10 is greater than the inner diameter of an o-ring in the Y-arm, and will therefore not pass through the o-ring. FIG. 1 shows the sleeve 10 prior to entry into a leg of a Y-arm port 60. In a currently preferred embodiment, the sleeve 10 is longer than the length of the Y-arm. FIG. 2 also illustrates a guide wire 65. Guide wires are well known in the art as one method of guiding the stent to its target site.

In both FIG. 1 and FIG. 2, one or more therapeutic agents may be in contact with polymeric coatings on coated stent 20. The system 100 may help treat, for example, heart disease, various cardiovascular ailments, and other vascular conditions by using catheter-deployed endovascular stents that have tailored polymeric coatings for controlling the timed-release properties of interdispersed or encased therapeutic agents. Treatment of vascular conditions may include the prevention or correction of various ailments and deficiencies associated with the cardiovascular system, the cerebrovascular system, urinogenital systems, biliary conduits, abdominal passageways and other biological vessels within the body.

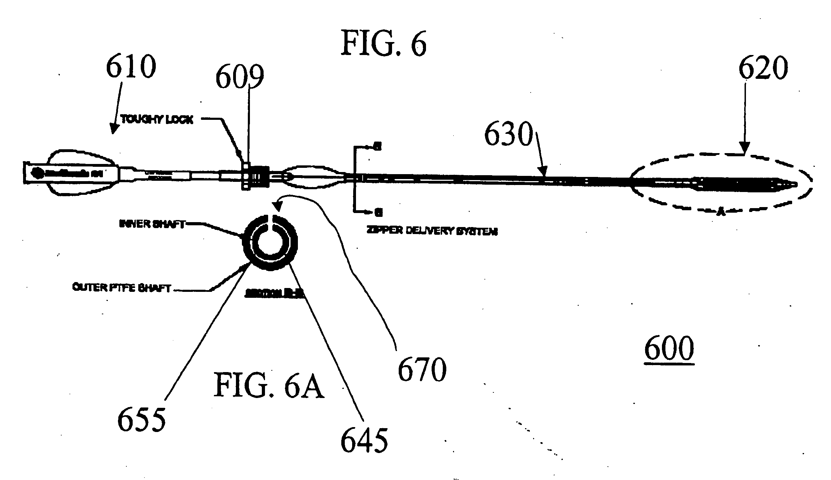

In FIGS 1 and 2, y-arm 60 generally provides a port to a vessel. Other ports into a vessel are possible, and this invention is adaptable to work with any port into a vessel. Other embodiments, some of which are illustrated in FIGS 6, 7, and 8, do not use a y-arm port, but use other ports that are generally known in the art.

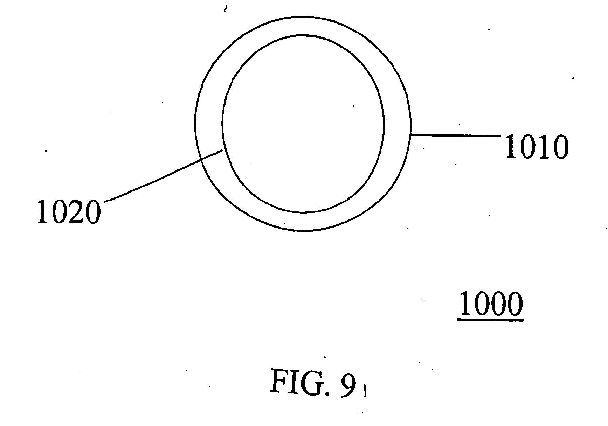

FIG. 9 depicts a cross section of the sleeve at 1000. The outer diameter of the sleeve is depicted as 1010, and the inner diameter of the sleeve is depicted as 1020. In each embodiment of the instant invention the sleeve generally comprises a hollow tube, although the inner and outer diameter vary along the length of the protective sleeve.

In each of the embodiments disclosed herein, the protective sleeve may comprise a lubricious covering, on the inner surface, the outer surface or both. Thus, referring to FIG. 10, a lubricious covering may cover at least a portion of outer surface 1010, inner surface 1020, or both inner surface 1010 and outer surface 1020. For example, a lubricious coating may be positioned on at least a portion of an outer surface of the protective sleeve. The lubricious coating may comprise a material such as phosphorylcholine, a hydrophilic coating, or a lubricious film. Other portions of the system disclosed herein may also be coated with lubricious coating to minimize the coefficient of friction between the coated surface and structures surrounding the coated surface. For example, a lubricious covering may be applied to the outer surface of the stent and the inner surface of the protective sleeve to ease removal of the protective sleeve at the target location. Patterns, stripes, and fractional coverage of the various surfaces with the lubricious coating may be used to control stent retention during delivery and deployment.

Generally, the outer diameter of the protective sleeve will be determined by determining whether the protective sleeve will be delivered to the vasculature, or whether the protective sleeve should remain in the port to the vessel. In the former case, the sleeve is allowed to pass through the o-ring, and in the second according to the present invention, the sleeve is not allowed to pass through the o-ring. The protective sleeve should have an inner diameter sized to allow free and unrestricted movement of the stent delivery assembly longitudinally, but with limited movement laterally. Optimally, the inner diameter of the sleeve will comprise a lubricious coating to provide for easier removal of the sleeve from the underlying stent delivery assembly. It may also be optimal to construct different sections of the sleeve from different materials. For example, the distal portion of the sleeve may comprise a material that has greater flexibility than the medial or proximal portion of the sleeve.

Where the sleeve has an outer diameter that is greater than the inner diameter of the toughy lock o-ring, the use of the catheter will be generally as used in the prior art. However, where the sleeve is to be removed in the vasculature, the system must also contain means to retract the sheath. Generally these means will be such that the sleeve may be pulled back, with an application of a retracting force at the proximal end of the sleeve. Such means are known in the art, and are in use for self-expanding stents. These means may include the use of wire or rod, or a tubular member. As these means are generally known in the art, the retraction means are not pictured in the figures. This invention is optimally used in conjunction with self-expanding stents, and the means for retracting the sleeve may be used in conjunction with the means for allowing the self-expanding stent to expand.

In the use of a self-expanding stent, it is common that the stent be prevented from expanding at an undesired location with the use of a sheath. Upon reaching the desired deployment site, these self-expanding sheaths are removed. Some embodiments of the protective sleeve disclosed herein may be coupled to a self-expanding stent sheath, although this is not required to practice the invention. Thus, some embodiments may operably connect the retraction means for retaining means of a self-expanding stent with the means to retract the protective sleeve disclosed herein.

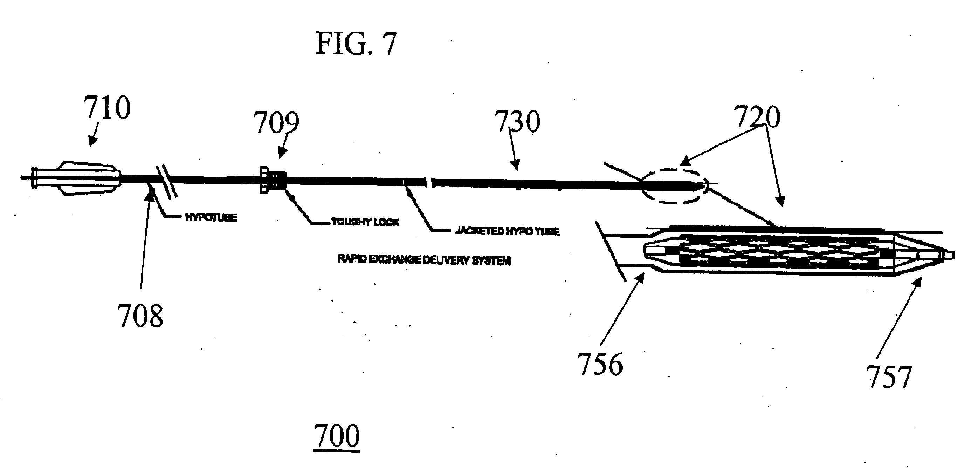

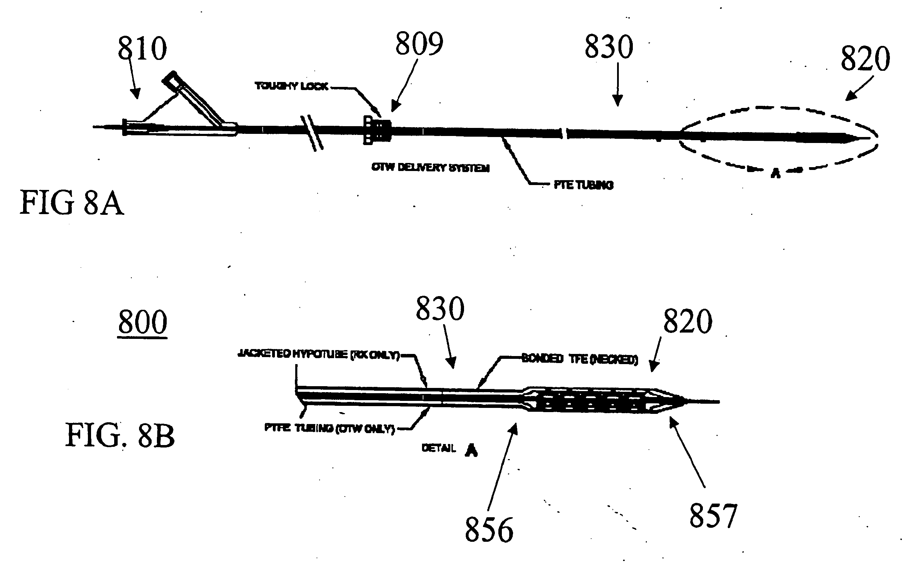

The sleeve is easily adapted to a variety of catheter delivery systems. Example adaptations of the sleeve are depicted in FIG. 6 (Zipper delivery), FIG. 7 (Rapid Exchange delivery), and FIG. 8A/8B (over the wire). This invention may be used to protect self-expanding stents.

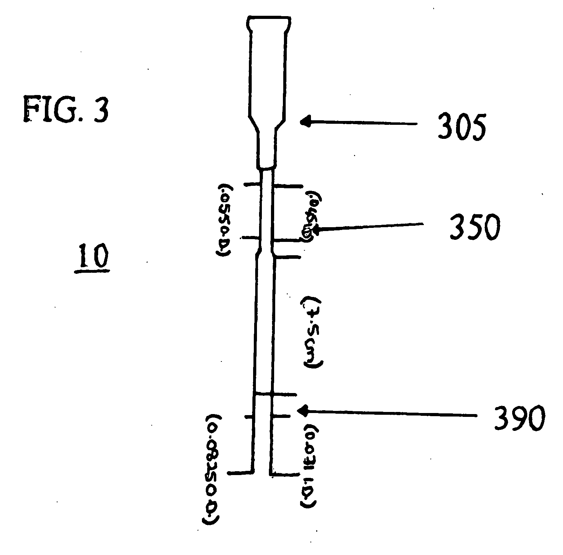

FIG. 3 shows a side view of the protective sleeve 10 in one embodiment of the instant invention. Proximal end 305 tapers to the medial portion 350. Medial portion 350 tapers to the distal end 390. In this embodiment, medial portion 350 has an inner diameter of .045 centimeters, and an outer diameter of .055 centimeters. The distal end 390 has an inner diameter of .071 centimeters, and an outer diameter of .0825 centimeters. These measurements are examples only, and any appropriate diameter may be used to practice the invention. In another example, the distal inner diameter will be sufficient to encircle an outer diameter of the catheter, the medial inner diameter will be sufficient to encircle the stent deployment assembly, and the proximal inner diameter will be sufficient to encircle the catheter.

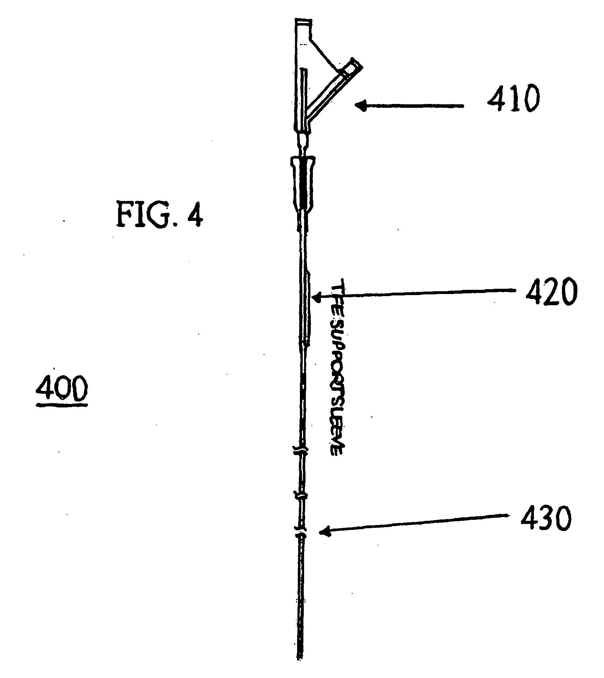

FIG. 4 illustrates stent assembly 400 as shipped. Stent assembly 400 comprises Y-arm 410, protective sleeve 420, and catheter 430. Stent assembly 400 is pictured with the sleeve 420 distal the Y-arm. Catheter 430 is shown surrounded by the sleeve 420.

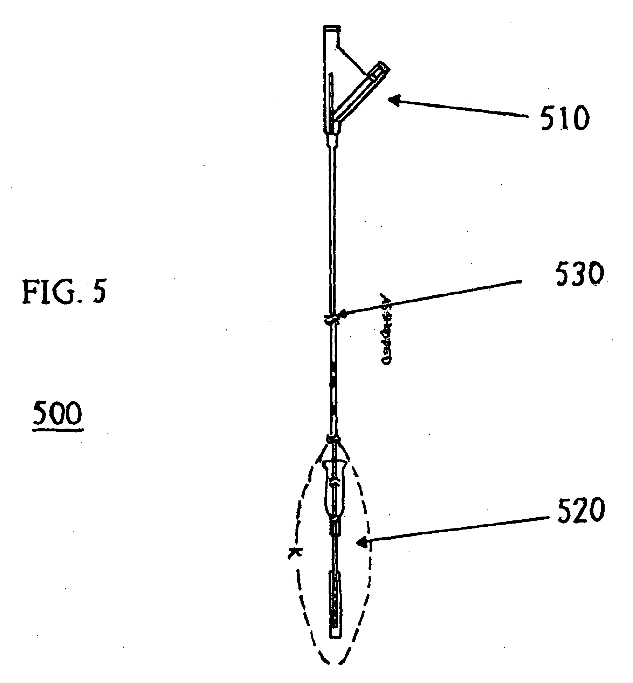

FIG. 5 shows the same embodiment as FIG. 4, but with the sleeve 520 in a deployed position. Stent assembly 500 comprises Y-arm 510, protective sleeve 520, and catheter 530. Sleeve 520 is now distal the position illustrated in FIG. 4, and continues to surround catheter 530. In zipper delivery systems, as illustrated in FIG. 6, the sleeve will comprise a guide wire notch, as is illustrated in greater detail below.

In FIG. 6 the sleeve 620 is adapted for use on a zipper delivery system. FIG. 6 shows stent assembly 600, comprising vessel port 610, toughy lock 609, protective sleeve 620, and catheter 630. FIG. 6A is a cross section of the sleeve 620 taken at line B-B. As can be clearly seen in FIG. 6A, when adapted for use on a zipper delivery system, the sleeve 620 comprises an inner shaft 645 and an outer shaft 655. Both the inner shaft 645 and the outer shaft 655 comprise a gap 670 to allow passage of the zipper. The zipper extends longitudinally through gap 670. Referring back to FIG. 6, toughy lock 609 comprises an o-ring (not shown). In embodiments of this invention, the o-ring will engage the outer diameter of the protective sleeve 620 and prevent the protective sleeve 620 from entering the vasculature. Catheter 630 extends longitudinally from the toughy lock 609, as is known in the art for zipper delivery systems. 610 is a port into a vessel that is not a y-arm.

In another embodiment, the system comprises a guide wire, and the protective sleeve comprises a guide wire notch. The guide wire extends longitudinally through the guide wire notch. The guide wire notch extends at least part of the distance from an outer surface of the protective sleeve through to the inner surface. The protective sleeve slides freely longitudinally along the guide wire riding in the guide wire notch, with substantially no lateral movement.

FIG. 7 illustrates an embodiment for a monorail system. FIG. 7 shows stent assembly 700, comprising vessel port 710, hypotube 708, toughy lock 709, protective sleeve 720, and catheter 730. Catheter 730 extends longitudinally from the hypotube 708, through the toughy lock 709. Protective sleeve 720 is pictured at the distal end of the catheter. The close-up view of the protective sleeve 720 shows the sleeve 720 tapering up at the proximal end 756 of the protective sleeve, and tapering down at the distal end 757 of the protective sleeve. 710 illustrates a port into a vessel that is not a y-arm. FIG. 7 shows an embodiment of the invention wherein the sleeve travels along the catheter to the stent deployment area, and therefore, the protective sleeve 720 is pictured distal the vessel port 710. In a monorail delivery system, the assembly 700 further includes a guidewire (not shown) and the protective sleeve 720 comprises a guidewire notch, wherein the assembly travels longitudinally along the guidewire disposed in the guidewire notch.

FIGS. 8 and 8A illustrate an embodiment utilizing an over-the-wire (OTW) delivery system. OTW systems are well known in the art. FIG. 8 shows stent assembly 800, comprising vessel port 810, toughy lock 809, protective sleeve 820, and catheter 830. FIG. 8A shows close-up view of the protective sleeve 820 shows the protective sleeve 820 tapering up at the proximal end 856 of the protective sleeve, and tapering down at the distal end 857 of the protective sleeve. FIG. 8 shows an embodiment of the invention wherein the sleeve travels along the catheter to the stent deployment area, and therefore, the protective sleeve 820 is pictured distal the vessel port 810.

## Claims

1. A system for treating a vascular condition, comprising:
a catheter (40);
a stent deployment assembly coupled to the catheter; the stent deployment assembly comprising a coated stent (20) including a stent framework and a drug coating (30) disposed on at least a portion of the stent framework; and
a protective sleeve (10) removably covering the stent deployment assembly and at least a portion of the catheter, wherein said sleeve comprises a hollow tube having a proximal outer diameter, a medial inner diameter, and a distal inner diameter; and wherein the distal inner diameter is sufficient to encircle an outer diameter of the stent deployment assembly, wherein the medial inner diameter is sufficient to encircle an outer diameter of the catheter, wherein the distal inner diameter is open, and wherein the protective sleeve is removed from covering the stent framework prior to deploying the stent; and **characterized by** a port to a vessel, the port (60, 610) including an O-ring having an O-ring inner diameter, wherein a proximal portion of the sleeve is positioned proximal to the port vessel and wherein the outer diameter of the proximal portion is greater than the O-ring diameter.

2. The system of claim 1 wherein the sleeve (10) comprises a material selected from the group consisting of nylon, polyurethane, polyethylene terephthalate, polyethylene, polytetrafluoroethylene, expanded polytetrafluoroethylene, an elastane, a thermoplastic elastomer, a woven polymeric fabric, or an expandable polymeric sheet.

3. The system of any preceding claim wherein the sleeve (10) comprises a material that dissolves while in a vasculature.

4. The system of any preceding claim further comprising:
a lubricious coating on at least a portion of a surface of the sleeve.

5. The system of claim 4 wherein the lubricious coating comprises a material selected from the group consisting of phosphorylcholine, a hydrophilic coating, and a lubricious film.

6. The system of any preceding claim wherein the sleeve (10) has a distal inner diameter of substantially .071 centimeters, a distal outer diameter of substantially .0825 centimeters, a medial inner diameter of .045 centimeters, and a medial outer diameter of .055 centimeters.

## Patentansprüche

1. System zur Behandlung eines vaskulären Zustands bzw. einer Gefäßerkrankung mit:
einem Katheter 840);
einer Stentabgabeanordnung, die mit dem Katheter gekoppelt ist; wobei die Stentabgabeanordnung einen beschichteten Stent (20) mit einem Stentrahmenwerk und einer auf wenigstens einem Abschnitt des Stentrahmenwerks angeordneten Medikamentenbeschichtung (30) aufweist; und
einer Schutzhülse (10), die die Stentabgabeanordnung und wenigstens einen Abschnitt des Katheters in entfernbarer Weise bedeckt, wobei die Hülse ein hohles Rohr mit einem proximalen Außendurchmesser, einem mittleren Innendurchmesser und einem distalen Innendurchmesser aufweist; und
wobei der distale Innendurchmesser ausreichend zum Umfangen bzw. Umgeben eines Außendurchmessers der Stentabgabeanordnung ist, wobei der mittlere Innendurchmesser ausreichend zum Umgeben eines Außendurchmessers des Katheters ist, wobei der distale Innendurchmesser offen ist, und wobei die Schutzhülse von der Bedeckung des Stentrahmenwerks vor der Abgabe des Stents entfernt wird; und **gekennzeichnet durch** einen Anschluss bzw. einen Port zu einem Gefäß, wobei der Port (60, 610) einen O-Ring mit einem O-Ring-Innendurchmesser beinhaltet, wobei ein proximaler Abschnitt der Hülse proximal des Portgefäßes angeordnet ist, und wobei der Außendurchmesser des proximalen Abschnitts größer als der O-Ring-Durchmesser ist.

2. System nach Anspruch 1, bei dem die Hülse (10) ein Material aufweist, das aus der Gruppe ausgewählt ist, die aus Nylon, Polyurethan, Polyethylen-Terephthalat, Polyethylen, Polytetrafluorethylen, expandiertem bzw. geschäumtem Polytetrafluorethylen, einem Elastan, einem thermoplastischen Elastomer, einem gewebten Polymergewebe oder einer expandierbaren bzw. schäumbaren Polymerschicht bzw. -platte besteht.

3. System nach einem der vorstehenden Ansprüche, bei dem die Hülse (10) ein Material aufweist, das sich auflöst, während es sich in einem Gefäß befindet.

4. System nach einem der vorstehenden Ansprüche, ferner mit:
einer schmierenden Beschichtung auf wenigstens einem Abschnitt einer Oberfläche der Hülse.

5. System nach Anspruch 4, bei dem die schmierende Beschichtung ein Material aufweist, das aus der Gruppe ausgewählt ist, das aus Phosphorylcholin, einer hydrophilen Beschichtung und einem schmierenden Film besteht.

6. System nach einem der vorstehenden Ansprüche, bei dem die Hülse (10) einen distalen Innendurchmesser von in wesentlichen 0,071 Zentimetern, einen distalen Außendurchmesser von im wesentlichen 0,0825 Zentimetern, einen mittleren Innendurchmesser von 0,045 Zentimetern und einen mittleren Außendurchmesser von 0,055 Zentimetern besitzt.

## Revendications

1. Système pour traiter un état vasculaire, comportant :
un cathéter (40) ;
un ensemble de déploiement d'endoprothèse ou stent couplé au cathéter ; l'ensemble de déploiement de cathéter comportant une endoprothèse enrobée (20) incluant une ossature d'endoprothèse et un enrobage de médicament (30) disposé sur au moins une partie de l'ossature d'endoprothèse ; et
un manchon protecteur (10) recouvrant de manière amovible l'ensemble de déploiement d'endoprothèse et au moins une partie du cathéter, dans lequel ledit manchon comporte un tube creux ayant un diamètre extérieur proximal, un diamètre intérieur médial et un diamètre intérieur distal ; et
dans lequel le diamètre intérieur distal est suffisant pour encercler un diamètre extérieur de l'ensemble de déploiement d'endoprothèse, dans lequel le diamètre intérieur médial est suffisant pour encercler un diamètre extérieur du cathéter, dans lequel le diamètre intérieur distal est ouvert, et dans lequel le manchon protecteur est empêché de recouvrir l'ossature d'endoprothèse avant le déploiement de l'endoprothèse ; et **caractérisé par** un orifice jusqu'à un vaisseau, l'orifice (60, 610) incluant un joint torique ayant un diamètre intérieur de joint torique, dans lequel une partie proximale du manchon est positionnée proximale au vaisseau d'orifice et dans lequel le diamètre extérieur de la partie proximale est plus grand que le diamètre de joint torique.

2. Système selon la revendication 1, dans lequel le manchon (10) comporte un matériau choisi parmi le groupe constitué de nylon, de polyuréthanne, de térephtalate de polyéthylène, de polyéthylène, de polytétrafluoroéthylène, de polytétrafluoroéthylène expansé, d'un élasthanne, d'un élastomère thermoplastique, d'un tissé polymérique ou d'une feuille polymérique expansible.

3. Système selon l'une quelconque des revendications précédentes, dans lequel le manchon (10) comporte un matériau qui se dissout lorsqu'il est dans un système vasculaire.

4. Système selon l'une quelconque des revendications précédentes, comportant en outre :
un revêtement lubrifiant sur au moins une partie d'une surface du manchon.

5. Système selon la revendication 4, dans lequel le revêtement lubrifiant comporte un matériau choisi parmi le groupe constitué de phosphorylcholine, d'un revêtement hydrophile et d'un film lubrifiant.

6. Système selon l'une quelconque des revendications précédentes, dans lequel le manchon (10) a un diamètre intérieur distal sensiblement égal à 0,071 centimètre, un diamètre extérieur distal sensiblement égal à 0,0825 centimètre, un diamètre intérieur médial de 0,045 centimètre, et un diamètre extérieur médial de 0,055 centimètre.
